# EUROPEAN PATENT SPECIFICATION

(11) **EP 2 827 838 B1**
(45) Date of publication and mention of the grant of the patent: **27.03.2019**
(21) Application number: 13710972.4
(22) Date of filing: 08.03.2013
(51) Int. Cl.: A61K 9/00, A61K 47/10, A61K 47/26, A61K 47/32, A61K 31/542

(54) **OPHTHALMIC PHARMACEUTICAL COMPOSITION CONTAINING A CARBONIC ANHYDRASE INHIBITOR AND METHOD FOR THE PREPARATION THEREOF**
OPHTHALMISCHE PHARMAZEUTISCHE ZUSAMMENSETZUNG MIT EINEM CARBOANHYDRASEHEMMER UND VERFAHREN ZUR HERSTELLUNG DAVON
COMPOSITION PHARMACEUTIQUE OPHTALMIQUE CONTENANT UN INHIBITEUR D'ANHYDRASE CARBONIQUE ET PROCÉDÉ POUR SA PRÉPARATION

(30) Priority: 22.03.2012 GR 20120100173
(43) Date of publication of application: 28.01.2015
(73) Proprietor: Pharmathen S.A., 15351 Pallini Attikis (GR)
(72) Inventor: KARAVAS, Evangelos, 15351 Pallini Attikis (GR); KOUTRIS, Efthimios, 15351 Pallini Attikis (GR); SAMARA, Vasiliki, 15351 Pallini Attikis (GR); MILOULI, Efstathia, 15351 Pallini Attikis (GR); KONTIZA, Ioanna, 15351 Pallini Attikis (GR); KOUTRI, Ioanna, 15351 Pallini Attikis (GR)
(86) International application number: PCT/EP2013/000697
(87) International publication number: WO 2013/139444

(56) References cited:
- WO-A1-98/25620
- WO-A1-2013/025696
- US-A1- 2006 257 487

## Description

### TECHNICAL FIELD OF THE INVENTION

The present invention relates to a process for the preparation of a stable ophthalmic pharmaceutical formulation comprising a therapeutically effective quantity of Brinzolamide or a pharmaceutical acceptable salt thereof and 0.01-0.05 w% of poloxamer 407 as a surfactant.

### BACKGROUNG OF THE INVENTION

Glaucoma is a disease, usually caused by high intraocular pressure, which leads to disruption of normal eye function and subsequently, degeneration of the eye. The damage can be extended to the optic nerve head and result in irreversible loss of the eyesight and if left untreated it could lead to irreversible blindness. Nowadays, it is believed by the majority of ophthalmologists that the increased intraocular pressure (also known as ocular hypertension) is the earliest phase in the onset of glaucoma. Later symptoms include optic nerve head damage and the characteristic glaucomatous visual effects.

The early methods for the treatment of glaucoma included the drug Pilocarpine, which produced undesired local side effects. More recently new regimes have been employed for the treatment of ocular hypertension and glaucoma.

It is known that carbonic anhydrase inhibitors are used for the treatment of ocular hypertension related to glaucoma. The drugs that belong to this family inhibit the enzyme carbonic anhydrase and thus, reduce the contribution of the aqueous humor formation made by the carbonic anhydrase pathway. However, these drugs cannot be used via a systemic route because then, they inhibit the enzymatic activity of carbonic anhydrase throughout the entire body. In general, the enzyme carbonic anhydrase plays a major role in regulating pH and fluid levels in the human body by converting carbon dioxide to carbonic acid and bicarbonate ions.

Targeting of the carbonic anhydrase inhibitor to the desired ocular tissue diminishes or even eliminates the side effects caused by the inhibition of carbonic anhydrase in the entire body, which can be as a severe as metabolic acidosis or less severe, like numbness, vomiting, tingling, general malaise and the like.

Brinzolamide, a carbonic anhydrase inhibitor, is the chemical molecule designated as (R)-4-ethylamino-3, 4-dihydro-2-(3-methoxy) propyl-2H-thieno[3,2-e]-1,2-thiazine-6-sulfonamide 1, 1 dioxide. It has been found to reduce intraocular pressure with fewer side effects compared to the earlier glaucoma treatments. Brinzolamide is a white to almost white powder with a melting point at 131°C. Furthermore, is insoluble in water and slightly soluble in alcohol and methanol.

Various methods are already known for the industrial preparation of dosage forms comprising a carbonic anhydrase inhibitor and especially Brinzolamide or salt thereof, as an active ingredient due to its useful therapeutical properties. However, according to prior art, substantial difficulties are encountered in the production of stable ophthalmic formulations due to the poor solubility of said active ingredient.

EP-B- 941 094 discloses a process for the preparation of Brinzolamide suspension and the use of Tyloxapol® and Triton® X-100 as a surfactant.

EP-A-2 394 637 discloses a process for the manufacture of sterile ophthalmic suspensions comprising Brinzolamide, characterized in that it comprises a step of sterilization of Brinzolamide by gamma irradiation or ethylene oxide.

Although each of the above patents represents an attempt to overcome the low aqueous solubility problems associated with topical pharmaceutical compositions comprising Brinzolamide, a need still exists for the development of a stable ophthalmic product that will overcome the deficiencies of the prior art.

### SUMMARY OF THE INVENTION

The present invention provides a method for the preparation of a stable ophthalmic formulation for topical administration comprising Brinzolamide or ophthalmological acceptable salts thereof, as the active ingredient, permitting adequate release of the active medicament that can be used for the treatment of ocular hypertension and glaucoma with improved pharmacotechnical characteristics of the composition and in particular adequate suspendability of the active ingredient within the finished dosage form.

A process for the preparation of a stable ophthalmic composition for topical administration for the treatment of ocular hypertension and glaucoma, comprising Brinzolamide or ophthalmologic acceptable salts thereof, as the active ingredient and 0.01 to 0.05 w% of Poloxamer 407 in order to provide adequate solubilization and stabilization of the low soluble active ingredient in the aqueous formulations is provided, wherein it comprises the following steps:
- First forming a solution of the surfactant in water for injection;
- Then adding to the solution the total quantity of Brinzolamide;
- Autoclaving the above mixture and immediately homogenize until ambient temperature is reached;
- Passing the solution through colloidal mill to reach the desired particle size;
- Subsequently, forming a second solution in water for injection by adding at least one tonicity agent, suspending agent, chelating agent and preservative agent and mixing until complete homogeneity;
- adjusting the pH of the second solution by adding NaOH or HCL;
- autoclaving the obtained solution and subsequently homogenize until ambient temperature is reached;
- Mixing gradually the two formed solutions until uniform, and
- Finally, adjusting the final mixture with water for injection and filling in appropriate container.

One preferred embodiment of the present invention is defined in dependent claim 2.

Other objects and advantages of the present invention will become apparent to those skilled in the art in view of the following detailed description.

### DETAILED DESCRIPTION OF THE INVENTION

A stable ophthalmic solution for topical administration needs to fullfill specific characteristics, for example pH value, osmolality, specific gravity and viscosity. Regulation of these characteristics through the selection of specific excipients will avoid any unwanted side effects such as visual blurring, burning sensation, low corneal contact and drying of the eye.

In particular, the viscosity of the ophthalmic solution should be regulated so as to benefit of increasing the ocular contact time, thereby increasing the drainage rate and increasing drug bioavailability. A secondary benefit is a lubricant effect that is noticeable to many patients. In addition, viscosity serves to retard the settling of the particles between uses and at the same time maintains their suspension for uniform dosing.

Further, an ophthalmic solution should meet the stability and the sterilization issues.

A major object of the present invention is to provide a stable pharmaceutical formulation for ophthalmic use containing Brinzolamide or ophthalmologically acceptable salts thereof. All pharmaceutical dosage forms should have impurities at very low levels, as said impurities might be toxic and harmful for the humans. Moreover, the impurities diminish the potency of pharmaceutical compositions during storage. They can also act as catalysts or intermediates in chemical reactions and change the drug into another form.

According to the present invention it has been found that a Brinzolamide ophthalmic composition is more stable and thus more potent when a surfactant, which is Poloxamer is used.

The surface or interfacial tension produced by polyethoxylated non-ionic surfactants at the concentration so that they form aggregates is usually higher compared to that of ionic surfactants, thus making the non-ionic surfactants less destructive on cell membranes and less irritating and toxic. In addition, the non-ionic surfactants are usually more bulky in size, less polar and less preferentially adsorbed at the surface, therefore, they have a tendency to associate together at a much lower concentration to reduce the surface free energy.

In addition, the concentration of Poloxamer 407 has been optimized, as when the concentration of a nonionic surfactant exceeds a certain level above the CMC (critical micelle concentration), the antimicrobial effectiveness of the preservative such as Benzalkonium Chloride is reduced. An effective quantity according to the present invention is from about 0.01% to 0.05% in the total volume of the finished dosage form.

Poloxamer is a non-ionic poly(ethylene oxide) (PEO)-poly(propylene oxide) (PPO) copolymer. It is used in pharmaceutical formulations as surfactant, emulsifying agent, solubilizing agent, dispersing agent, and in vivo absorbance enhancer. It is available in several grades differing in molecular weight and composition of the hydrophilic PEO block (a) and hydrophobic PPO block (b). In addition, the concentration of Poloxamer in the final drug product is significantly lower than the maximum reported level in the FDA Inactive Ingredient List (IIG) for ophthalmic suspension, which ensures its safe use in the final drug product under ophthalmic application.

A process for the preparation of a stable ophthalmic composition for topical administration for the treatment of ocular hypertension and glaucoma, comprising Brinzolamide or ophthalmologic acceptable salts thereof, as the active ingredient and 0.01-0.05w% of Poloxamer 407 in order to provide adequate solubilization and stabilization of the low soluble active ingredient in the aqueous formulations, is provided, wherein it comprises the following steps:
- Fist forming a solution of the surfactant in water for injection;
- Then adding to the solution the total quantity of Brinzolamide;
- Autoclaving the above mixture and immediately homogenize until ambient temperature is reached;
- Passing the solution through colloidal mill to reach the desirable particle size;
- Subsequently, forming a second solution in water for injection by adding at least one tonicity agent, suspending agent, chelating agent and preservative agent and mixing until complete homogeneity;
- adjusting the pH of the second solution and autoclaving;
- Mixing the two solutions until uniform and;
- Finally, adjusting the volume with water for injection and filling in appropriate containers.

The pharmaceutical composition of the present invention may also contain one or more additional formulation ingredients selected from a wide variety of excipients. According to the desired properties of the composition, any number of ingredients may be selected, alone or in combination, based upon their known uses in preparation of stable dosage ophthalmic compositions.

Such ingredients may include, but are not limited to ophthalmologically acceptable carriers, osmotic agents, antibacterials, buffering agents, viscosity enhancing agents, tonicity, chelating and solubilizing agents. Any optional excipients must be compatible with Brinzolamide or ophthalmologically acceptable salts thereof, so that they do not interfere with the active ingredient in the composition.

Osmotic agents may be, for example, mannitol, dextrose anhydrous, dextrose hydrous, glycerin, potassium chloride, sodium chloride.

Carriers may be selected from water, water miscible solvents such as lower alkanols or aralkanols, vegetable oils, polyalylene glycols, carboxymethylcellulose, isopropyl myristate and the like.

Antibacterial agents may be selected from thimerosal, benzalconium chloride, methyl and propyl paraben, benzyl alcohol, benzyl dodecinium bromide and phenylthanol.

Buffering or pH adjusting agents may be selected form sodium hydroxide, hydrochloric acid, sodium chloride, sodium borate, sodium acetate, sodium citrate, gluconate buffers, sodium phosphate, sodium dihydrogen phosphate, disodium hydrogen phosphate potassium phosphate, potassium dihydrogen phosphate, dipotassium hydrogen phosphate, sodium borate, potassium borate, sodium citrate, disodium citrate, sodium acetate, potassium acetate, sodium carbonate, sodium hydrogen carbonate and trometamol.

Suspending agents may be selected from carboxyvinyl polymers (Carbomers), hydroxypropyl methyl cellulose, hydroxyethyl cellulose, polyvinyl alcohol, polyvinyl pyrrolidone, xanthan gum, guar gum and dextrans.

Tonicity agents may be selected from sodium chloride, potassium chloride, calcium chloride, propylene glycol, glycerol, glycerin, polyethylene glycol, magnesium chloride and the like.

Chelating agents may be, for example EDTA and solubilizing agents such as Cremophor EL and tween 80.

All percentages stated herein are weight percentages based on total composition weight, unless otherwise stated.

The following examples illustrate a preferred embodiment in accordance with the present invention.

### EXAMPLES

### Example 1: (composition 5 is according to the invention)

**TABLE 1: Qualitive & quantitative formula of compositions 1 to 5**

| **Ingredients** | **Composition 1** | **Composition 2** | **Composition 3** | **Composition 4** | **Composition 5** |
|---|---|---|---|---|---|
| | **Quantity per 5ml suspension (mg)** | | | | |
| **Mixture A** | | | | | |
| Brinzolamide | **50.00** | **50.00** | **50.00** | **50.00** | **50.00** |
| Cremophor® RH40 | 1.25 | | | | |
| Cremophor® EL | | 1.25 | | | |
| Polysorbate 80 | | | 1.25 | | |
| Poloxamer 188 | | | | 1.25 | |
| Poloxamer 407 | | | | | 1.25 |

| **Mixture B** | | | | | |
|---|---|---|---|---|---|
| Mannitol | 165.00 | 165.00 | 165.00 | 165.00 | 165.00 |
| Sodium Chloride | 12.50 | 12.50 | 12.50 | 12.50 | 12.50 |
| Edetate disodium | 0.50 | 0.50 | 0.50 | 0.50 | 0.50 |
| Carbomer 974P | 23.25 | 23.25 | 23.25 | 23.25 | 23.25 |
| Benzalkonium Chloride solution 50% w/v | 0.98 | 0.98 | 0.98 | 0.98 | 0.98 |
| NaOH / HCl | qs to pH 7.5 | qs to pH 7.5 | qs to pH 7.5 | qs to pH 7.5 | qs to pH 7.5 |
| water for injection (ml) | qs to 5.0ml | qs to 5.0ml | qs to 5.0ml | qs to 5.0ml | qs to 5.0ml |
| **Total volume (ml)** | **5.0ml** | **5.0ml** | **5.0ml** | **5.0ml** | **5.0ml** |

Compositions 1 to 5 with different surfactant agent are prepared. The exact formula of the five compositions is shown in table 1.

All five compositions were prepared by using the same manufacturing process.

Initially, a first mixture is prepared by dilution of the surfactant in water and subsequently Brinzolamide is added therein. A second mixture is prepared by dissolving mannitol in water and subsequently dissolving sodium chloride, edetate sodium, carbomer 974P and benzalconium chloride therein. The pH value of the second mixture is measured and adjusted by adding the necessary amount of NaOH or HCl.

Finally, the two mixtures are homogenized together and the volume is adjusted by the necessary amount of water.

Compositions 1 to 5 were compared in terms of sedimentation volume and resuspendability as well as their chemical stability. The sedimentation volume was determined by keeping 50 ml of each suspensions in stoppered measuring cylinder and stored undisturbed at room temperature. The separation of clear liquid was noted at intervals of 5 days up to 45 days. The sedimentation volume was calculated using the formula Vu/Vo, where Vu is the volume of sediment and Vo is the original volume (50ml) of each composition tested. Values close to 1 where the sediment volume is almost equal to the original volume of each composition tested indicate a stable suspension.

**TABLE 2: Sedimentation volume of compositions 1-5.**

| | **Vu/Vo 5 days** | **Vu/Vo 15 days** | **Vu/Vo 25 days** | **Vu/Vo 35 days** | **Vu/Vo 45 days** |
|---|---|---|---|---|---|
| **Composition 1 (Cremophor RH-40)** | 0,75 | 0,72 | 0,7 | 0,69 | 0,68 |
| **Composition 2 (Cremophor EL)** | 0,75 | 0,71 | 0,71 | 0,69 | 0,69 |
| **Composition 3 (Polysorbate 80)** | 0,77 | 0,74 | 0,73 | 0,73 | 0,7 |
| **Composition 4 (Poloxamer 188)** | 0,96 | 0,93 | 0,91 | 0,9 | 0,89 |
| **Composition 5 (Poloxamer 407)** | 0,99 | 0,97 | 0,95 | 0,93 | 0,92 |

The results show that compositions 4 and 5 with Poloxamer are the most stable suspensions.

In order to test the resuspendability of compositions 1 to 5, accelerated settling studies were performed by subjecting 9 ml of the composition in a separate 15ml glass tube to centrifugation for 20 minutes at 1000 rpm. After centrifugation, the composition was caused to spin (40 rpm) on a rotor. The resuspendability of the settled material was tested by measuring the time required to resuspend the sediment completely (NMT 15 seconds).

**TABLE 3: Resuspension time of compositions 1 to 5.**

| **Composition** | **1** | **2** | **3** | **4** | **5** |
|---|---|---|---|---|---|
| **Resuspension Time (Seconds)** | 45 | 41 | 43 | 14 | 14 |

As it is shown in the table, composition 4 and 5 with Poloxamer have the best resuspendability since the resuspension time is less than 15 seconds.

Regarding the chemical stability, it is obvious that compositions 4 and 5 with Poloxamer as surfactant are the most stable after 6 months storage at 25°C/ 60% RH, 30°C/ 60% RH and 40°C/ 75% RH.

**TABLE 4: Stability data of compositions 1-5 after 6 months at 25°C/ 60% RH, 30°C/ 60% RH and 40°C/ 75% RH.**

| **Composition** | **1** | | | **2** | | | **3** | | | **4** | | | **5** | | |
|---|---|---|---|---|---|---|---|---|---|---|---|---|---|---|---|
| **Storage temperature (°C)** | **25** | **30** | **40** | **25** | **30** | **40** | **25** | **30** | **40** | **25** | **30** | **40** | **25** | **30** | **40°C** |
| **Impurity A (NMT 1.50%)** | 1,06 | 1,58 | 2,12 | 0,80 | 1,59 | 1,91 | 0,98 | 1,78 | 2,17 | 0,48 | 1,06 | 1,23 | 0,46 | 1,01 | 1,21 |
| **Total Impurities (NMT 2.0%)** | 0,29 | 0,33 | 0,39 | 0,31 | 0,33 | 0,39 | 0,32 | 0,34 | 1,08 | 0,35 | 0,52 | 0,34 | 0,27 | 0,34 | 0,37 |

The physical characteristics such as pH value, osmolality, viscosity and specific gravity of all five compositions were satisfying. However, especially with regard to composition 1, 2 and 3 after 24 hours the active pharmaceutical ingredient was separated from the other ingredients and sedimentation of Brinzolamide was observed.

The use of poloxamer as a surfactant (compositions 4 & 5) significantly improved the solubilization of Brinzolamide in aqueous composition, especially the use of poloxamer 407 (composition 5) where the impurities levels are lower.

### Example 2:

Composition 5 containing Poloxamer 407 as a surfactant provided satisfactory results and said composition was prepared following the same manufacturing process as stated in example 1. Steam sterilization (autoclave) has been used as the sterilization process in Composition 5.

Sterilization according to example 1 of the present invention has been performed after the final homogenization of mixtures A and B. Although physical characteristics are acceptable, degradation products have been increased over the accepted limits. Furthermore, due to the solubility of the active ingredient at autoclaving temperatures, large needle-like crystals have been formed on cooling down of the final formulation, which settle as sediment and have been difficult to be resuspended.

Composition 5 according to example 2 of the present invention has been sterilized, wherein mixture A and B have been autoclaved separately. After sterilization of mixture A, Brinzolamide has been separated from water creating a biphasic mixture. In particular, the upper phase was the water and the lower was the melted Brinzolamide which on cooling down of the mixture A converted to a compact solid mass. As a result, mixing of Mixture A and Solution B was impossible.

Accordingly, the sterilization process has been amended, wherein mixture A and mixture B of composition 5 have been autoclaved separately and immediately after sterilization, hot mixture A at temperature about 60°C - 70°C has been homogenized and added gradually to hot mixture B at temperature about 60°C - 70°C.

The physical characteristics of composition 5 thus sterilized are satisfactory and the degradation product is within accepted limits. However, some large particles have been observed which were difficult to be resuspended.

Therefore, according to example 2 the following sterilization process has been conducted to composition 5: mixture A and mixture B of composition 5 have been autoclaved separately and mixture A immediately after its sterilization has been homogenized until ambient temperature and then said mixture A has been mixed with ambient temperature mixture B, which mixture B has been homogenized, as well. Physical characteristics of composition 5 thus sterilized are satisfactory and Brinzolamide is well suspended.

### Example 3

Composition 5 of example 1 using the sterilization process of example 2 has been tested in a large scale production and in order to obtain a well suspended product, mixture A passed through a colloid mill until the particle size is less than about 20 µm.

**TABLE 5: Qualitive & quantitative of composition 5 according to the present invention**

| **Ingredients** | **Quantity per 5ml suspension (mg)** |
|---|---|
| **Brinzolamide** | **50.00** |
| Mannitol | 165.00 |
| Poloxamer 407 | 1.25 |
| Sodium Chloride | 12.50 |
| Edetate disodium | 0.50 |
| Carbomer 974P | 23.25 |
| Benzalkonium Chloride solution 50% w/v | 0.98 |
| NaOH / HCl | qs to pH 7.5 |
| water for injection (ml) | qs to 5.0ml |
| | |
| **Total volume (ml)** | **5.0** |

Composition 5 has been prepared by using the following manufacturing process: formation of a first mixture A by diluting Poloxamer 407 in water and subsequently adding Brinzolamide therein. Mixture A is sterilized in an autoclave and then is stirred fiercely until homogeneity and ambient temperature is reached. Mixture A is transferred and passed through a colloidal mill until its particle size is less than about 20 microns.

A second mixture B is formed by dissolving Mannitol in water. Sodium chloride is added in said solution and dissolved. Then, Edetate disodium is added in the obtained mixture and dissolved. Subsequently, Carbomer 974P is added and when dissolved, Benzalkonium chloride is also added and dissolved under stirring.

Subsequently, the pH value of the obtained mixture B is being adjusted by addition of the appropriate amount of NaOH or HCl. Mixture B is also sterilized in an autoclave and stirred until homogeneity and ambient temperature is reached.

Finally, mixture A is added gradually to mixture B and mixed. The final mixture is adjusted with the necessary amount of water and the product is stored in an appropriate container.

Physical characteristics of composition 5 are satisfactory, particle size results are acceptable and stability results are adequate as presented in table 6 below.

**TABLE 6: Stability data after 3 months storage at 25°C/ 60% RH and 30°C / 60%**

| **Control tests** | **Limits** | **Stability data after 3 months** | |
|---|---|---|---|
| | | 25°C/ 60% RH | 30°C/ 60% RH |
| Specific Gravity | 1.010-1.020g/ml | 1.019 | 1.018 |
| pH | pH = 7.1-7.9 | 7.4 | 7.42 |
| Viscosity | 440 ± 85cp | 511 | 512 |
| Osmolality | 270-320 mOsmol/kg | 292 | 288 |
| Assay | 95.0-105.0% of the stated amount of Brinzolamide | 99.2% | 99.0% |
| Benzalkonium Chloride Assay | 85.0-115.0% of the stated amount of Benzalkonium Chloride | 99.5% | 99.1% |
| EDTA Assay | 85.0-115.0% of the stated amount of EDTA | 96.0% | 97.7% |
| Related Substances of Brinzolamide | Methane Sulfonyl Impurity NMT 0.15% | 0.08% | 0.08% |
| | Isopropyl impurity NMT 0.15% | ND | ND |
| | Impurity B NMT 0.30% | 0.14% | 0.14% |
| | Any Individual Unknown NMT 0.50%, | ND | ND |
| | Total NMT 2.0% | 0.22% | 0.22% |
| Enantiomeric Purity | Related Compound A NMT 1.50% | 0.35% | 0.36% |

The bioequivalence and efficacy of composition 5 according to the present invention has been tested and confirmed that all requirements have been fulfilled.

## Claims

1. A process for the preparation of a stable ophthalmic composition for topical administration for the treatment of ocular hypertension and glaucoma, comprising Brinzolamide or ophthalmologic acceptable salts thereof, as the active ingredient and from 0.01% to 0.05% by weight based on the total weight of the composition of surfactant poloxamer 407, in order to provide adequate solubilization and stabilization of the low soluble active ingredient in the aqueous formulations, wherein it comprises the following steps:
- First forming a solution of the surfactant in water for injection;
- Then adding to the solution the total quantity of Brinzolamide;
- Autoclaving the above mixture and subsequently homogenize until ambient temperature is reached;
- Passing the obtained solution through colloidal mill to reach the desired particle size;
- Subsequently, forming a second solution in water for injection by adding at least one osmotic agent, a tonicity agent, a suspending agent, a chelating agent and a preservative agent and mixing until complete homogeneity;
- adjusting the pH of the second solution by adding NaOH or HCl;
- autoclaving the obtained solution and subsequently homogenize until ambient temperature is reached;
- Mixing gradually the two formed solutions until uniform, and
- Finally, adjusting the final mixture with water for injection and filling in appropriate container.

2. The process according to claim 1, wherein the composition comprises also mannitol, benzalkonium chloride, edetate disodium, sodium chloride and carbomer.

## Patentansprüche

1. Ein Verfahren zur Herstellung einer stabilen ophthalmischen Zusammensetzung zur topischen Verabreichung zur Behandlung von Augenhypertonie und Glaukom, umfassend Brinzolamid oder ophthalmologische akzeptable Salze davon als Wirkstoff und 0,01 bis 0,05 Gew .-%, bezogen auf das Gesamtgewicht der Zusammensetzung des Tensids Poloxamer 407, um eine ausreichende Auflösung und Stabilisierung des schwerlöslichen Wirkstoffs in den wässrigen Formulierungen bereitzustellen, wobei er die folgenden Schritte umfasst:
- einer Lösung des Tensids in Wasser zur Injektion bilden;
- der Gesamtmenge Brinzolamid zur Lösung hinzufügen;
- der obigen Mischung autoklavieren und anschließendes bis zur Umgebungstemperatur homogenisieren;
- der erhaltenen Lösung durch eine Kolloidmühle leiten, um die gewünschte Teilchengröße zu erreichen;
- anschließend einer zweiten Lösung in Wasser zur Injektion durch Zugabe mindestens eines osmotischen Mittels, eines Tonizitätsmittels, eines Suspendiermittels, eines Chelatbildners und eines Konservierungsmittels bilden und bis zur vollständigen Homogenität mischen;
- des pH-Wertes der zweiten Lösung durch Zugabe von NaOH oder HCl einstellen;
- der erhaltenen Lösung autoklavieren und anschließendes bis zur Umgebungstemperatur homogenisieren;
- allmähliches der beiden gebildeten Lösungen bis zur Gleichförmigkeit mischen und
- Zum Schluss wird die endgültige Mischung mit Wasser für die Injektion eingestellt und in einen geeigneten Behälter gefüllt.

2. Verfahren nach Anspruch 1; wobei die Zusammensetzung Mannitol, Benzalkoniumchlorid, Edetatdinatrium, Natriumchlorid und Carbomer umfasst.

## Revendications

1. Un procédé pour la préparation d'une composition ophtalmique stable pour administration topique pour le traitement de l'hypertension oculaire et du glaucome, comprenant le Brinzolamide ou un sel ophtalmologique acceptable de celui-ci, en tant que principe actif et de 0.01% à 0.05% en poids, basé sur le poids total de la composition, de surfactant poloxamer 407, afin d'assurer la solubilisation et la stabilisation adéquates du principe actif peu soluble dans les formulations aqueuses, comprenant les étapes suivantes:
- premièrement, former une solution de surfactant dans de l'eau pour injection;
- puis ajouter à la solution, la quantité totale de Brinzolamide;
- autoclaver le mélange ci-dessus et par la suite, homogénéiser jusqu'à ce que la température ambiante soit atteinte;
- passer la solution obtenue dans un moulin colloïdal pour atteindre la taille désirée de particules;
- par la suite, former une deuxième solution dans de l'eau pour injection en ajoutant au moins un agent osmotique, un agent de tonicité, un agent de suspension, un agent chélateur et un agent de conservation et mélanger jusqu'à homogénéité complète;
- ajuster le pH de la deuxième solution en ajoutant NaOH ou HCl;
- autoclaver la solution obtenue et par la suite, homogénéiser jusqu'à atteindre la température ambiante;
- mélanger graduellement les deux solutions formées jusqu'à uniformité; et
- finalement, ajuster le mélange final avec de l'eau pour injection et remplir dans un contenant approprié.

2. Le procédé selon la revendication 1, dans lequel la composition comprend en outre du mannitol, du chlorure de benzalkonium, de l'édétate disodique, du chlorure de sodium et du carbomer.
